# EUROPEAN PATENT APPLICATION

(11) **EP 3 236 716 A1**
(43) Date of publication of application: **25.10.2017**
(21) Application number: 17166548.2
(22) Date of filing: 13.04.2017
(51) Int. Cl.: H05B 37/02, F21V 14/00, A61B 90/30

(54) **OPERATING ROOM LIGHTING SYSTEM AND METHOD FOR PRESENTING ILLUMINATION ADJUSTMENT INSTRUCTIONS TO AN OPERATOR OF THE OPERATING ROOM LIGHTING SYSTEM**

(30) Priority: 15.04.2016 FI 20165332
(71) Applicant: Merivaara Oy, 15150 Lahti (FI)
(72) Inventor: NIEMINEN, Jyrki, 15700 Lahti (FI)
(74) Representative: Berggren Oy, Helsinki & Oulu

(57) **Abstract**

An operating room lighting system, which comprises a lighthead support (10) for a lighthead (1) with a plurality of light elements (2), especially LED light elements, for generating a light field on an object (9) to be illuminated, as well as adjustment elements (4) for controlling features of the lighting system, especially for controlling features which modify a light field generated by the lighthead (1), said adjustment elements (4) comprising a portable pointing device (4; 2) for controlling the lighting system by means of a pointing beam (221) by the action of a lighting system operator. In the invention, the portable pointing device (2) further comprises projection elements (29) for projecting adjustment instructions (5) on or around a light field (271) generated by the pointing device (2) and/or on a light field generable or generated by the lighthead.

## Description

The invention relates to an operating room lighting system according to the preamble of claim 1.

The invention relates also to a method according to the preamble of claim 18 for presenting illumination adjustment instructions to an operator of the operating room lighting system.

The operating device for an operating room lighthead, such as a portable pointing device, can be equipped with various user input elements such as buttons for adjusting a desired light field feature, such as the intensity of a light field or the shape of a light field, by the action of a user him-/herself. In connection with the user input elements of pointing devices, there may have been produced, e.g. by methods of printing technology, operating instructions such as a visual indication concerning a feature adjustable by means of the elements, e.g. the text 'light field intensity', and another indication about an actual difference made in said feature by operating the elements. For example, a symbolic instruction '+', produced in connection with a button or pushbutton, indicates to the user that the adjustable feature increases in value by pressing the button, whereas a '-' button indicates that the feature decreases in value respectively, typically by a predetermined numerical value.

In some working situations, however, these types of conventional adjustment practices have proved inconvenient or at least non-optimal for adjusting a portable pointing device, especially if there are a multitude of adjustable features. Because of its way of being operated, the portable pointing device must be maintained relatively small in terms of its physical size, and in case the operation-indicating instructions are placed in connection with pushbuttons, the set of instructions easily becomes, among other things, difficult to read because of small print. In the event of a large number of pushbuttons, the adjustment instructions additionally become easily unclear and/or difficult to understand. Using an adjustment element solely by memorized instructions and then for performing the actual adjustment procedure is in turn highly inconvenient. It is a result of these deficiencies in the clarity of adjustment instructions for a portable pointing device that, when implemented in a traditional way, finding a correct portable pointing device adjustment may present a challenge.

Especially in a medical working environment, such as in an operating or examination room environment, it is impractical from the standpoint of a treatment procedure or diagnosis performing person to turn his/her eyes away from an object under treatment or diagnosis, e.g. from human tissue or more generally a patient, in order to perform a desired lighthead adjustment with a pointing device. Especially when performing a surgical procedure, it may also be hazardous if the surgeon, for example during an on-going surgical operation, would have to take his/her eyes off the surgical site. When using currently generally employed remote controller-operated lightheads, it is nevertheless necessary for the lighthead operator to focus his/her eyes on the actual remote controller in order to see symbols provided for example in the vicinity of a pushbutton. In this case, when adjusting the illumination, the surgeon's head may be in a different position than during the performance of a surgical operation, whereby the illumination also possibly becomes adjusted incorrectly and the adjustment procedure may have to be frequently repeated in order to provide a desired final value for the adjustable feature. It is an objective of the invention to at least alleviate the above-described shortcomings of prior art solutions.

Accordingly, it is a principal objective of the invention to provide a portable pointing device for an operating room, wherein the features of an operating room lighthead, especially the features of a light field, relating to an adjustment or the like regarding each button of a user input device, are presented with clarity.

It is a further objective of the invention to provide a lighting system for operating room service, whose portable pointing device can be used for presenting illumination-related adjustment instructions and also for adjusting features of the operating room lighthead's light field in such a way that the lighting system operator need not disengage his/her eyes from the site of illumination.

Another objective of the invention is to provide a portable pointing device for an operating room, wherein a feature of the operating room lighthead, relating to each button or the like of a user input device, can be checked in a simple manner.

Still another objective of the invention is to present the features of an operating room lighthead, relating to an adjustment or the like regarding each button of a user input device, in such a way that the size and the manner of presenting the instructions are as clear as possible from the user's standpoint.

These objectives are attained with an operating room lighting system of claim 1, as well as with a method of claim 18 for presenting illumination adjustment instructions to an operator the operating room lighting system.

More specifically, the invention of claim 1 relates to an operating room lighting system, which comprises a lighthead support for a lighthead with a plurality of light elements, especially LED light elements, for generating a light field on an object of surgical operation to be illuminated on the patient, as well as adjustment elements for controlling features of the lighting system, especially for controlling features of a light field generated by the lighthead, said adjustment elements comprising a portable pointing device for controlling the lighting system by means of a pointing beam of said pointing device by the action of a lighting system operator. The portable pointing device further comprises projection elements for projecting, on or around a pointing device-generated pointing beam, adjustment instructions relating to a change in one or more features of a lighthead-generated light field or to a change in the light field's location. The portable pointing device has projection elements comprising at least one laser light source, preferably a semiconductor laser, for projecting said adjustment instructions, as well as at least one light-transmitting optical film which is adapted to provide an adjustment instruction from light generated by the at least one laser light source, said optical film comprising diffractive optical microstructures such as surface-relief structures and/or embedded structures for producing the adjustment instructions.

In particular, the invention relates to a lighting system of claim 1, wherein the light field of a lighthead is focusable on a site in the patient submitted to a medical procedure.

More specifically, the invention relates also to a method for presenting illumination adjustment instructions to the operator of an operating room lighting system, said lighting system comprising a lighthead with a plurality of light elements, especially LED light elements, for generating a light field on an object of surgical operation to be illuminated on a patient, as well as adjustment elements for controlling features of the lighting system, especially for controlling features of a light field generated by the lighting system's lighthead, said adjustment elements comprising a portable pointing device for controlling the lighting system by means of a pointing beam of said pointing device by the action of a lighting system operator. The method comprises projecting adjustment instructions relating to controlling features of the lighting system, especially projecting, on or around a pointing beam generated by the pointing device, adjustment instructions relating to a change in one or more features of a light field generated by the lighting system's lighthead or to a change in the light field's location. The projecting is carried out by means of at least one laser light source, preferably a semiconductor laser, and at least one light-transmitting optical film in such a way that the light of said laser light source is conducted through the optical film, preferably a diffractive film, for producing adjustment instruction patterns.

The adjustment instructions refer in this context to adjustment instructions pertaining to changes in features of a light field generated by a lighting system, particularly by a lighthead of the lighting system, or to changes in a light field's location. The adjustment instructions pertain to operating the user input elements of a pointing device, i.e. are explicitly operating instructions for user input elements. The adjustment instructions may also refer to features pertinent to a specific switch, button or touch-sensitive area of the user input element or to how said switch, button or touch-sensitive area of the user input element must be operated for changing the light field of a lighting system, especially that of a lighthead.

The adjustment instructions preferably comprise adjustment instruction patterns, which apply to features of a lighthead's light field that are adjustable or achievable with user input elements of a pointing device.

In this context, the user input element refers to a switch, button or touch-sensitive area, whose flipping, pressing or touching enables selecting or adjusting a controllable feature of the light field of a lighting system, especially that of a lighthead.

The adjustment instruction pattern(s) is (are) perceived in this context as pictograms, logograms, and words presentable in visual form.

The adjustment instruction is produced within the light beam of a pointing device or in its immediate vicinity, i.e. immediately around the light beam. In the event that an adjustment instruction is produced immediately around the light beam, the exact location of the adjustment instruction will be determined on the basis of how the projection elements and the laser light source are disposed relative to each other in the pointing device.

Major benefits are attained by the invention with respect to operating room pointing devices of the prior art. The invention provides several merits over the prior art and these merits vary according to embodiments of the invention.

It is a basic idea of the invention to project the adjustment instructions, affixed to pushbuttons or the like of a pointing device, within or around a light beam generated by the pointing device for adjusting features of a light field generated by a lighthead or generable by a lighthead.

Thereby is attained a considerable benefit in that the adjustment elements can be used not only for adjusting each selected or currently "active" feature as indicated by instructions, such as by turning the elements or a component thereof, e.g. a handle, in a specific direction (which can be indicated by an arrow), but also for selecting a feature subjected to adjustment from among a plurality of features.

The operator may also assure him-/herself about an active feature to be adjusted at a given time by identifying adjustment instructions, which are visible at that particular moment and which have been designed in the best possible way to be inherently associable with the discussed feature. For example, the adjustment instructions for color temperature may comprise a symbol 'K', whereby the skilled person knows immediately that an adjustment of color temperature (K->Kelvin, unit for color temperature) is in question. Another benefit provided by this is that the user does not inadvertently adjust a wrong feature of the lighthead's light field, which might lead to even serious consequences because of the working environment (operating room).

When using an operating room lighting system of the invention, the operator of an operating room lighthead may obtain adjustment instructions relating to the features of lighting and can also perform the adjustment of lighting without having to take his/her eyes off an object of illumination, such as a patient. Thus, for example repositioning of the operator's head does not result in the necessity of adjusting the lighting several times for the reason that lighting is adjusted incorrectly as the effect of a head shadow cannot be effectively taken into account as lighting is being adjusted. Another major benefit attained by the invention is that, because the adjustment instructions are produced on a light field outside the pointing device, the presentation environment no longer imposes physical limitations to the shape of instructions or to the size and presentation mode of displayed symbols. The size of symbols and letters can be maintained larger and the presentation mode thereof can be kept considerably clearer as the instructions are displayed on a to-be-illuminated object (patient) present on the operating table with respect to the situation in which the same instructions would be presented in the vicinity of the buttons of a pointing device. This clarifies considerably the readability of adjustment instructions.

In an operating room environment, the employed optics may also be selected in such a manner that, when the light field of a lighthead is intended to be focused on an area of the patient subjected to a medical procedure, the adjustment instructions are produced around or outside this light field as the area is pointed by a pointing device. Thereby is gained the advantage that the projected adjustment instructions do not fall for example upon a surgical site. Hence, the procedure, for example a surgical operation, is also not disturbed in any way as a result of presenting the adjustment instructions.

The invention provides also other significant further benefits, which relate to various embodiments of the invention.

In the invention, the pointing device has its projection elements adapted to project adjustment instructions as a regularly repeating pattern or structure within or around a pointing beam generated by the pointing device. The projection elements comprise a laser light source, preferably a semiconductor laser, as well as at least one light-transmitting optical film which is adapted to produce an adjustment instruction from light generated by said at least one laser light source. The adjustment instruction is preferably an adjustment instruction pattern, i.e. a visually presentable picto- or logogram.

When adjustment instructions are provided by using a designated special light source or sources, such as semiconductor lasers, the instructions can be projected as sharply as possible as well as distinctively and be thereby absorbed by the user in an easy-to-read manner e.g. even in conditions of bright background lighting.

In some embodiments, optionally those of fixed optics, it is possible to implement different instructions by means of different light sources. E.g. the light of a first laser can be used for producing first instructions and the light of a second laser can be used for producing second instructions. The light beams of the lasers fall preferably upon different locations in the optics of projection elements, such as in a film which is light-transmitting yet contains optical light-controlling or -modulating surface and/or embedded structures.

By projecting the adjustment instructions for a light field feature as a repeating pattern or structure, which is readable or identifiable from various directions, e.g. in the form of a circle around a light beam, especially a pointing beam produced by a pointing device, the operator is able to read the instructions easily from various angles and positions without essentially changing his/her location or position from the original, possibly optimal working position or location.

The presentation of adjustment instructions explicitly within or around a light field produced by a pointing device provides an ability to enhance the distinctiveness of adjustment instructions from the surroundings.

The present invention and benefits attainable thereby will now be illustrated in even more detail with reference to the accompanying figures. Hence:
Fig. 1 shows, from a direct side view, one lighting system of the invention.
Fig. 2 shows schematically functions as well as physical components present in a pointing device for an adjustment element of the lighting system of fig. 1.
Fig. 3 shows schematically how the physical components of a pointing device adjustment element for the lighting system of fig. 1 are disposed within the pointing device.
Fig. 4 shows still schematically, from a direct side view, the generation of adjustment instructions relating to functions of a pointing device.
Figs. 5A-5F show various adjustment instructions.
Fig. 6 shows schematically, in a block diagram, a method of the invention in its principal aspects.

Next will be briefly reviewed first the aspects of the invention presented in each figure, as well as principal features and structures of the invention presented in the figures.

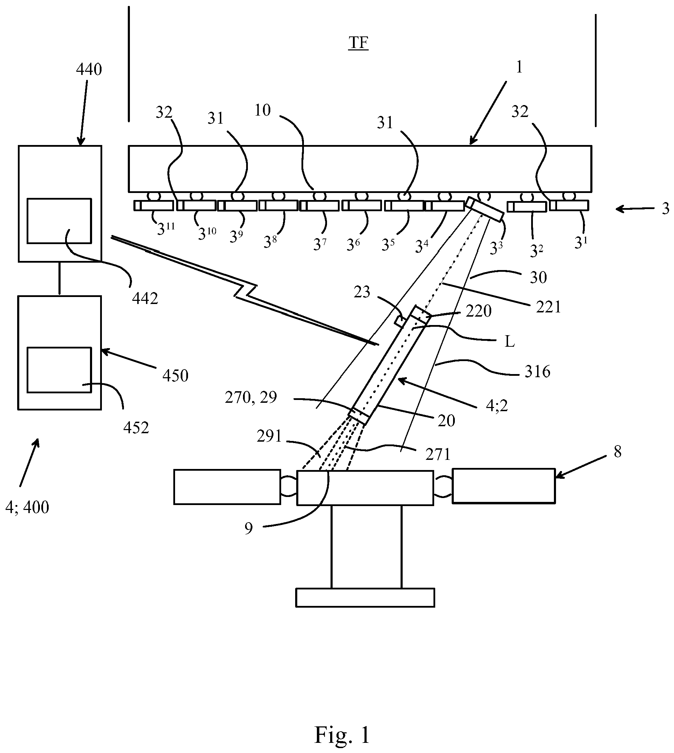

A lighthead 1 shown in fig. 1 comprises a plurality of light elements 3¹, 3², 3³.. 3¹¹ installed on a lighting support 10 of the lighthead. Each light element comprises at least one light diode or LED and necessary optics as well as observation elements 32, for example light sensors, which are adapted to detect a pointing beam 221, which arrives from a light diode unit 220 of a pointing device 2 and which is co-directional with a longitudinal axis L of the pointing device's 1 body 20. The lighthead 1 is located in an inlet air frame TF for incoming fresh air, the inlet air passing therefrom onto an operating table 8.

Fig. 1 shows adjustment elements 4 for the lighthead 1, including a control unit 440 for a control system 400, observation elements 32 and/or an observation unit 450, as well as a portable pointing device 2. The operator uses the pointing device 2, whose first end is provided with a light diode unit 27 intended for pointing at an object and whose second end is provided with a light diode, laser or IR unit 22, to determine those light elements 3¹, 3², 3³.. 3¹¹ of the lighthead 1 which participate in the illumination of an object, for example a patient present on the operating table 8.

The observation elements 32 may have been implemented also some other way, for example by mounting the same directly on a frame of the lighthead 1. The observation elements 32 may have been implemented in several ways, for example by mounting the same directly into engagement with the lighthead 1 or with the observation unit 450 of the control system 400 as depicted in fig. 1. The lighthead 1 includes additionally only partially visible motion mechanisms 31, which are controlled by the control unit 440 and by means of which it is possible to manipulate the light elements 3¹, 3², 3³.. 3¹¹.

In a preferred embodiment of the invention, the control unit 440 of the control system 400 and its data transfer unit 442 are capable jointly with the observation elements 32 of determining the light elements 3 to be used for illumination.

The pointing device 2 according to the invention 1 comprises at least one disposable or rechargeable battery 24, which functions as a power source and is located in the body 20 of the pointing device 2 so as to enable its replacement or recharging by way of a separate charging device or a charging interface (not shown) included in the pointing device, at least one position sensor 25 for determining the orientation, a data transfer unit 26 which comprises at least an RF and/or IR transmitter for communicating position data plus other information to the illumination control unit in a wireless manner. The data transfer unit 26 may comprise alternatively an RF and/or IR transmitter/receiver, whereby software items of the pointing device 2 can be updated over a wireless communication from the control unit or from some other computer. The pointing device 2 further comprises a light diode unit 27 comprising at least one light diode for facilitating an alignment of the pointing device by means of a light beam produced thereby. The pointing device 2 may also comprise a second light diode unit 22 comprising at least one light diode, and a laser/IR transmitter which is located at a second end of the elongated pointing device 2 and by means of which is determined one or more light elements to be used for illuminating an object.

Thus, the operating room lighting system comprises a lighting support 10 for the lighthead 1, having a plurality of light elements 2, especially LED light elements, for generating a light field on an object 9 to be illuminated, as well as adjustment elements 4 for controlling features of the lighting system, particularly for controlling features which modify a light field produced by the lighthead 1. The adjustment elements 4 comprise a portable pointing device 2 for controlling the lighting system by means of a pointing beam 221 with the actions of an operator. The portable pointing device 2 further comprises projection elements 29 for projecting adjustment instructions 5 within or around a light beam generated by the pointing device 2 and/or the lighthead.

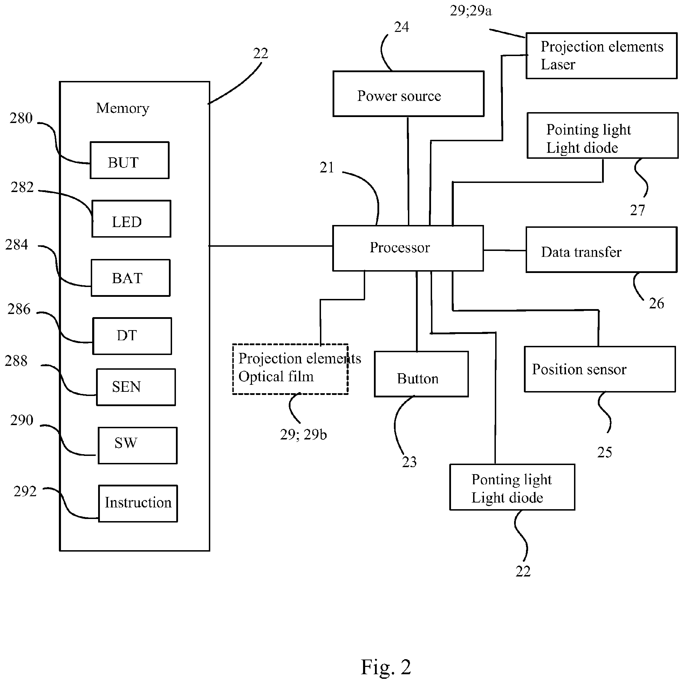

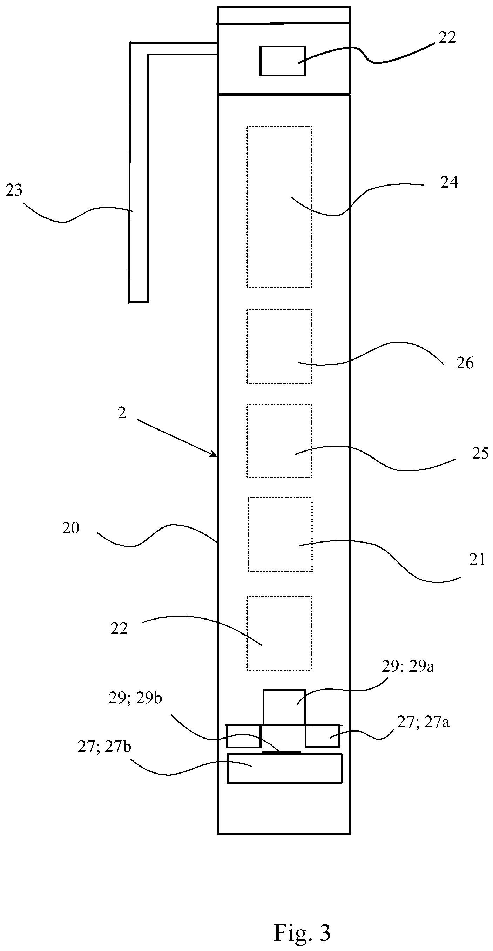

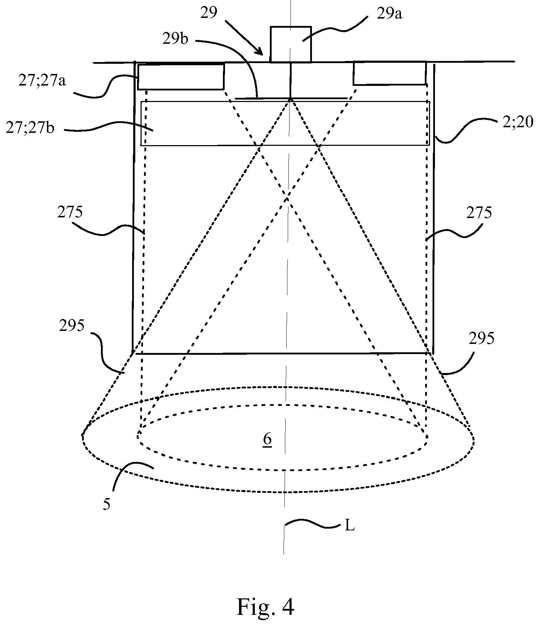

It is in figs. 2, 3 and 4 that a more detailed view is depicted of the portable pointing device 2, i.e. a so-called light pen, inside whose cylindrical body 20 is packed a processor 21, a memory unit 220, a user input button 23, which is implemented as a function button mounted on a side of the pointing device's 2 body 20 (cf. figs. 1 and 3) and by whose pressing is produced an illumination command, the intensity of illumination being determined from the duration of pressing, a replaceable battery 24, a position sensor unit 25, and a data transfer unit 26 equipped with an RF transmitter.

The pointing device 2 has its first end provided with a light beam or pointing beam 271, which is intended for indicating a surgical site 9 on a patient and which is produced by light diodes 27, 27a of the light diode unit 27 and by its optics 27, 27b.

The pointing device 2 further comprises a second light diode, laser or IR unit 22, which is mounted on a second end of the pointing device 2 as seen from the light diodes which produce the pointing beam 271. It is possible to use this second unit by producing a pointing beam 221 to determine light elements 3¹, 3², 3³... used for the illumination of an object.

The pointing device's memory (unit) 220 further comprises a program 280 controlling operation of the at least one function button 23, a program 282 controlling operation of the at least one light diode unit 27 of the pointing light, a program 284 controlling operation of the at least one battery or other power source 24, a program 286 controlling operation of the data transfer unit 26, a program 288 controlling operation of the at least one position sensor 25, a program 290 used by the pointing device 2 to control the lighthead unit, as well as a program 293 used for controlling a laser 29, 29a of the projection elements and in some cases also the optical film or elements affixed thereto, such as a mask or an optical film conveying motor (not shown in the figures).

The projection elements 29 of the pointing device 2 comprise a film 29; 29b, which is preferably permeable to light at its desired wavelengths, such as within a range of visible light, and which is preferably optically essentially transparent (e.g. light transmission of at least 80%, 85% or 90%). The film can be e.g. a flexible plastic film such as a polymer film. The film may contain e.g. polycarbonate or PMMA (polymethylmethacrylate).

The film 29b is optically functional. It can be adapted to produce an adjustment instruction pattern from the light generated by at least one light source 29a comprised in the projection elements 29. For the purpose, the optical film 29b may comprise refractive and/or diffractive surface-relief structures e.g. in micrometer level size class or even smaller and/or e.g. (subsurface) structures either laminated or otherwise embedded in the multilayer structure. These light-conducting or -modulating structures may comprise lattice grooves, other recesses, ridges, bulges, etc. In cross-section, the structures may comprise various sharp or rounder shapes such as triangular shapes, parallelogram shapes, rectangular shapes, trapezoidal shapes, etc. The film 29b may contain a number of optical masks.

For each adjustment instruction pattern the film 29b may comprise a number of dedicated light-conducting/modulating structures or e.g. structure groups including several structures positioned physically in the proximity of each other.

Optionally, the film 29b or a respective separate film can be adapted to conduct, modulate or at least to allow light through itself also from other light sources 27b or light source groups provided for example by the light diode unit 220.

The film 29b can be e.g. less than 2mm, preferably less than 1 mm or 0.5mm in thickness.

In connection with the film 29b can be disposed, optionally cast e.g. by injection molding with the film 29b serving as an insert, or laminated e.g. by means of pressure, an adhesive and/or pressure, a sheet protecting the optics of the lighthead 1 and preferably comprising an optically essentially transparent material, such as a plastic or glass sheet 27b. The sheet 27b can have a scratch-resistant, moisture-repellent and/or antibacterial surface (coating).

In some embodiments, at least some of the light sources 27a, 29a can be at least partially embedded in the optical film 29b or in the sheet 27b for protecting the sources and/or for improving optical coupling between themselves and the film/sheet 29b, 27b. Preferably, however, the light source 29a of the projection elements 29 comprises one or more lasers or other pointing light sources 29a.

The employed light sources 27a, 29a can be surface mount components and/or produced with printed electronics methods, e.g. by inkjet printing or silk-screen printing with reference e.g. to OLED sources (organic LED).

The light source 29a for presenting adjustment instructions 5 may comprise a number or a plurality of laser light sources such as semiconductor lasers. These light sources 29a can be explicitly adapted to the presentation of adjustment instructions.

In the case of several light sources 29a, an individual light source can be dedicated (exclusively adapted) to project some specific adjustment instruction pattern. Respectively, in order to project this particular pattern, the film 29a may contain dedicated optical structures as indeed pointed out above.

The pointing device 2 can have the optics 27b of its light diode unit 27 and the projection elements 29 can have the optical film 29b thereof accompanied also with movable elements, such as translatorily and/or rotatably movable elements. These elements can be optical, such as masks, lenses and/or mirrors, which enable modification of an optical transmission pathway extending from the light sources 27a, 29a by way of the optics 27b or the optical film 29b to an object 9 to be pointed with the pointing device or illuminated with the lighthead.

The lighthead 1 comprises also a plurality of other elements, not shown in the figure for the sake of clarity. These elements include e.g. a frame, articulation parts, connecting parts for attachment e.g. to ceiling structures or other support members. The elements may contain e.g. plastics or metal.

The lighthead 1 may include one or more distance sensors 25 for example for detecting obstacles in an optical transmission path between the lighthead 1 and the object 9 of illumination. Upon detecting an obstacle, the light elements 3 or individual light sources can be controlled with the pointing device 2 accordingly, in the simplest case switched on or off, so as to compensate for a difference made by the obstacle in the lighting of an object. Optionally, the distance sensor 25 can be used for measuring a distance between the lighthead 1 and the object 9, on the basis of which e.g. the intensity of lighting can be adjusted optionally so as to keep it constant.

In the portable pointing device 2 according to the described embodiment, a computer program 290 stored in its memory unit 22 is adapted, jointly with at least the processor 21, to bring the pointing device 2 at least to determine a position of the pointing device 2, intended for focusing the lighting, with at least one position sensor 25 so as to provide position data and to transmit the position data by means of the data transfer unit 26 to a control unit intended for controlling the lighting unit, which controls the lighting unit in such a way that the lighting unit produces lighting to the object from a direction determined by the position data and by location data, said location data having been established by determining a location of the portable pointing device 2 with respect to the lighting unit.

In one embodiment for a pointing device 2, as described in any of the preceding embodiments, the pointing device 2 has its position determined with at least one acceleration sensor 25 and with at least one processor 21.

The pointing device 2 according to another embodiment is adapted to determine, by means of at least one processor 21, the intensity of lighting on the basis of a command for producing lighting intensity data received by way of the control button 23 intended for its adjustment.

The pointing device 2 according to yet another embodiment is adapted to determine, by means of at least one processor 21 and a laser rangefinder (not shown in the figures), its distance to an object of illumination for producing distance data.

The pointing device 2 according to one embodiment is adapted to determine, by means of at least one processor 21, the size and/or shape of an area to be illuminated on the basis of a command received by way of the control button 23 intended for the adjustment thereof and on the basis of position data of the pointing device 2 for producing areal data.

The pointing device 2 according to one embodiment is adapted to determine, by means of at least one processor 21, the color temperature of lighting on the basis of a command for producing color temperature data received by way of the control button 23 intended for its adjustment.

The pointing device 2 according to one embodiment is adapted to transmit, by means of the data transfer unit 26, in a wireless manner, along with the position data, to the control unit at least one of the following: lighting intensity data, pointing device distance data from an object to be illuminated, light field areal data and color temperature data, and the lighting unit will be controlled by the control unit on the basis of data received by the latter.

The lighting unit's computer program 290 according to one embodiment, by means of which the lighting is controlled with the portable pointing device 2 and which is executed with the processor 21, comprises a determination code for determining a position of the pointing device 2 intended for focusing the lighting and for producing position data, and a transmission code for transmitting the position data to a control unit, which is intended for controlling the lighting unit and which controls the lighthead 1 in such a way that the lighthead 1 generates lighting for the object 9 of illumination from a direction determined by the position data and location data, said location data having been established by determining a location of the pointing device 2 with respect to the lighting unit 1.

According to one embodiment, the lighting unit's computer program 290 further comprises a determination code for determining the intensity of lighting and for producing intensity data.

According to one embodiment, the computer program 290 further comprises a determination code for determining the distance to an object to be illuminated and for producing distance data.

According to one embodiment, the computer program 290 further comprises a determination code for determining the size and/or shape of an area to be illuminated and for producing areal data. The adjustment instructions consistent with the shape of an area to be illuminated or a light field can be presented by means of the projection elements 29 of the pointing device 1, cf. fig. 55-55F, as subsequently described.

According to one embodiment, the computer program 290 further comprises a determination code for determining the color temperature of lighting and for producing color temperature data.

The lighting unit's computer program 290 can also be implemented in such a way that, instead of or in addition to color temperature, it is possible to adjust, by means of a determination code, at least one other optical feature of lighting, such as for example increasing and/or decreasing the light of a specific spectral range with respect to the rest of the light, for example accentuating red or some other color as necessary.

The computer program 290 according to one embodiment further comprises a transmission code for transmitting the position data in a wireless manner to the control unit jointly with at least one of the following: intensity data, distance data, areal data and color temperature data, the lighting unit being controlled by said control unit on the basis of data received by the latter.

In the computer program 290 according to one embodiment, the discussed computer program is a computer program product which comprises a computer-readable communication medium provided with a computer program code intended to be executed with a computer.

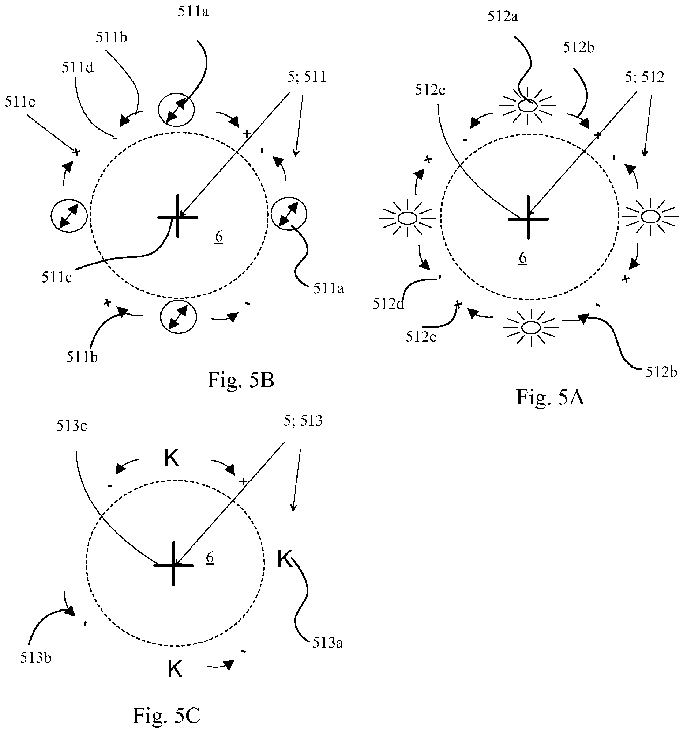

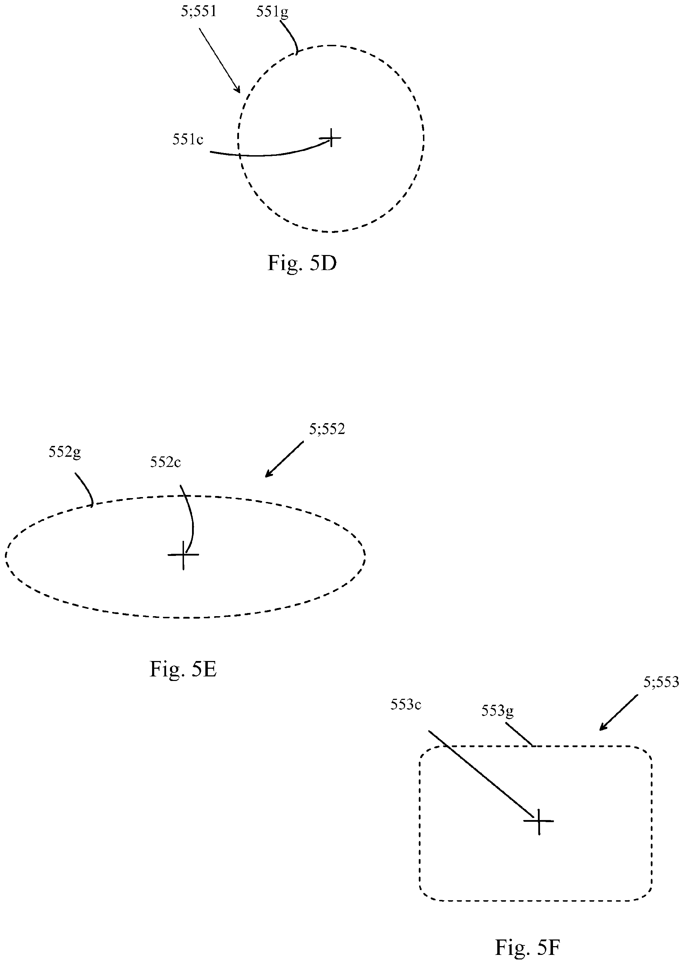

The projection elements 29 of the pointing device 2 can be used for producing adjustment instructions 5 within the pointing device's pointing beam 271 or especially within an instruction beam 291 present around the same. The projection elements 29 are used for projecting, onto or especially around the pointing beam 271, various adjustment instructions 5 for operating the user input elements 23 of the pointing device 2. These embodiments of adjustment instructions 5 achievable with the projection elements 29 are illustrated in figs. 5A-5E.

Figs. 5D-5C show a focusing/aiming pattern produced by certain embodiments of a lighthead according to the invention, such as a point or more preferably an easier-to-detect, more complex shaped and/or larger pattern such as a cross 551 c, 552c, 553c. In addition, the pattern may but need not include e.g. a circumferential, such as circular, elliptical or edgewise rounded square-shaped indicator 551 g, 552g or 553g, such as a line or dashed line, which demonstrates e.g. an essential size/diameter/shape and location (e.g. the edge with respect to a boundary value selected for the lighting intensity) of the light field.

It is by means of the pattern 551, 552 or 553 that the orientation of a light field is easy to ensure and, if necessary, to change as desired e.g. by placing the cross 551 c, 552c, 553c on top of the midpoint of a site of surgical operation.

Fig. 5A shows one embodiment for first projectable adjustment instructions. A sun symbol 512A can be used for indicating to the user that e.g. a brightness adjustment is in question. Technically, the (perceived) brightness of a target can be increased by means of the lighthead 1 e.g. by increasing the volume of a light stream generated by light sources included in the light elements 3.

In relation to the adjustment of e.g. said brightness but also other features, a '+' symbol 512e and a curved arrow 512b pointing thereto indicate that the adjustable feature, such as brightness, increases by turning for example the user input button 23 of the pointing device 2 of the adjustment elements 4 in the designated direction.

A '-' symbol 512e and a curved arrow 512b pointing thereto indicate that the adjustable feature decreases by turning for example the user input button 23 of the pointing device of the adjustment elements 4 in the designated direction.

Fig. 5B shows a second embodiment for second projectable adjustment instructions, which preferably differ from said first instructions shown in fig. 5A in terms of e.g. projected patterns, their size, location, brightness, color or the like. The adjustment of a light field feature relating to the second instructions, such as color temperature 511A, takes place by utilizing preferably the same button 23 of the pointing device.

Again, a '+' symbol 511 e and a curved arrow 511 b pointing thereto indicate that the adjustable feature, such as brightness, increases by turning for example the user input button 23 of the pointing device of the adjustment elements 4 in the designated direction.

A '-' symbol 511 e and a curved arrow 511 b pointing thereto indicate that the adjustable feature decreases by turning for example the user input button 23 of the pointing device of the adjustment elements 4 in the designated direction.

Between adjustments of features such as brightness 600 and temperature 700 can be alternated by means of a user interface not shown here for the button 23.

Fig. 5C shows one embodiment for third projectable adjustment instructions relating e.g. to the adjustment of an area of illumination, such as the adjustment of a size, diameter and/or shape. The same way as the sun symbol 512a referring to the first brightness adjustment instruction, the symbol 'K' (Kelvin) 513a is demonstrative and easy to understand for a skilled person as a symbol of color temperature adjustment, the double-headed arrow inside a circle is likewise respectively highly suitable for the demonstration of adjustment relating to the dimensions of an area. Of course, instead of or in addition to symbols, the adjustable feature could be visualized in projection e.g. by means of text and/or numerals.

In another embodiment according to the invention (not shown by a figure), the lighting system comprises a lighthead connected by way of a pivotable spring arm to operating room ceiling structures and comprising a plurality of light elements present on a single mounting support 10 and provided with one or more light diodes, as well as a protective dome surrounding these light elements, and each light element of the lighting system comprises a light diode or LED, said light element being installed at a certain point of the mounting support, whereby the light diode is coupled to orienting means of the adjustment elements as well as to optics capable of being pivoted by said orienting means, the orienting means being coupled functionally to a control unit for controlling the optics of each light element.

In another embodiment according to the invention (not shown by a figure), the lighting system comprises a mounting plane, which is constructed from a frame fitted with a plurality of elongated rod type mounting supports, said mounting supports being provided with a plurality of light elements comprising at least one light diode or LED, whereby the mounting brackets are arranged in two groups, each of said groups including at least two mounting brackets, the first group of which is set on a first level and the second group on a second level such that the mounting brackets of each group are apart or spaced from each other and that the first group's mounting brackets lie at an angle, most preferably at a right angle, relative to the second group's mounting brackets, and the lighting support, along with its frame and mounting supports, is arranged in the proximity of the ceiling of an operating room or the like facility essentially above the operating table so as to be located below a supply air frame TF of the facility to be illuminated.

Next described will be operation of the portable pointing device 2 in general and then explicitly generation of the adjustment instructions 5 in the pointing device 2.

The pointing device 2 has general operation as follows: when pointing to a to-be-illuminated object (not shown) on the operating table 8 with a light beam or pointing beam 271 starting from the light diode unit 27, the user activates from a function button 23, present for example on a side of the pointing device 2, a light diode unit 220 which generates a light beam or pointing beam 221 which is oriented towards the light elements. The pointing beam 221 is co-directional with the object-pointing light beam or pointing beam 271 and the longitudinal axis L of the pointing device's 2 body 20.

The observation elements 32 identify a light element 3 towards or closest to which the pointing beam 221 produced by the light diode unit 220 falls in the lighthead 1 and communicate a message about it to the control unit 400; 440. On the basis of a pointing message about the light element 3, position data received thereby according to the preceding example, and possibly other information intended for controlling the lighting, as well as location data, the control unit 440 determines with how many light elements 3 and in which way they shall illuminate the object 9. In the case of fig. 1, the illumination is provided by a light element 3³ upon which the pointing beam 221 falls, whereby the control unit 440 pivots by means of motion mechanisms 31 the light element 3³ in such a way that the illumination occurs co-directionally with the pointing device's 2 body 20. If necessary, the illumination can be enhanced for example by means of neighboring light elements 3⁴ and 3².

Respectively, the operator can use the pointing device 2 to define boundaries for an area to be illuminated, whereby the observation elements 32 identify which light elements 3 the pointing beam 221 falls upon as the area boundaries are being defined. In the event that, in the process of defining the area, the pointing beam 221 falls upon light elements 3⁴ and 3², the illumination can be effected by using just those or, alternatively, all the light elements 3¹, 3², 3⁴ designated by the pointing beam 21 and fallen inside the area defined thereby.

In some embodiments, the lighthead 1 can be capable of being configured by an operator, fitter or installer, or some other person in terms of functions of the lighthead such as adjustable features and/or adjustment interval. E.g. the sequence of adjustable features can be modifiable in terms of features to be included therein and/or the order thereof, especially if the adjustable feature is chosen by making use of a user input element, such as the button 23 and a selector, which is common thereto. Configuration can be conducted by means the lighthead's user interface or e.g. a separate maintenance interface.

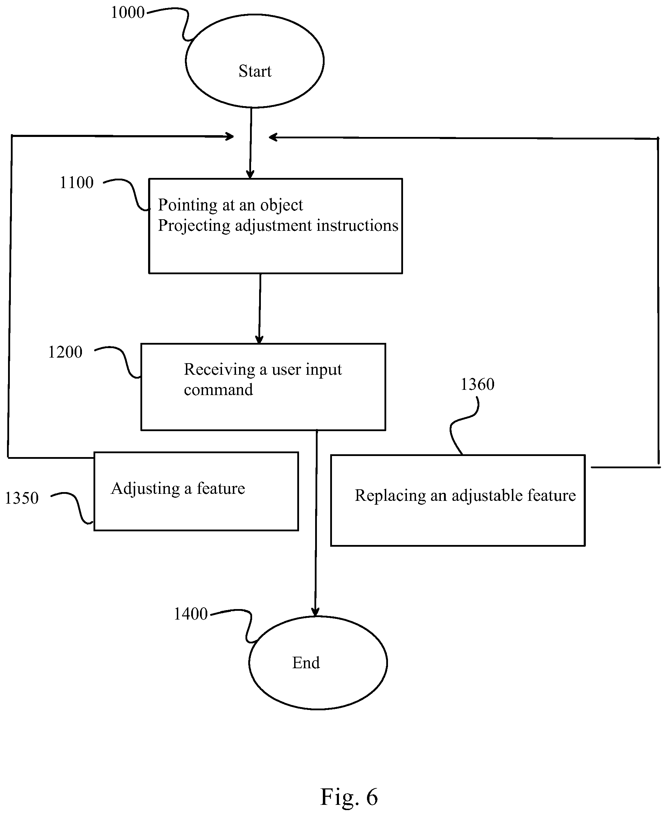

The projection of adjustment instructions will now be described in even more detail with reference to fig. 6.

Step 1100 comprises pointing at an object, such as a patient, with a portable pointing device 2. Adjustment instructions 5 are projected by means of projection elements 29 of the pointing device 2 either along the boundaries of a light field 6 generated by the pointing device or on the light field itself. Spot lighting is conducted according to present adjustment values, such as e.g. brightness or color temperature. In case of wishing to display with the pointing device 2 for example the shape of a light field, the light field of the lighthead 1 has not yet been necessarily established at this point, whereby the adjustment instructions 5 will be projected on what will become a light field of the lighthead.

Step 1200 comprises receiving a command from the user by way of the lighthead's user input element 23. The command can be an adjustment-related command, such as a replacement/selection command for an adjustable (active) feature of the lighthead's light field or an adjustment command for a feature already in active state, typically an adjustment value increasing or decreasing command. The user interface may comprise a number of user input elements also for purposes other than for switching the lighthead 1 (or lighting) on and off.

When the command is an adjustment command, there is executed in the next step at 1350 the adjustment of an adjustable feature in response to the command. For example, turning the button 23 for a certain time and/or to a certain extent may represent a change in the adjustment value of a feature in proportion to the time and/or the extent of turning the handle (rotation angle). Alternatively, a single rotating action may accomplish a preselected change in the adjustment value regardless of the time or the extent of rotation.

When the command is a replacement/selection command received by way of the user input elements 23 such as a selector, there is executed at step 1350 the replacement of each feature, which is adjustable through a user interface by means of user input elements 23, on the basis of a selection logic programmed in the lighthead by proceeding, e.g. in the adjustment sequence, from a current feature (e.g. color temperature) to the next (e.g. brightness).

Execution of the method is brought to an end at step 1400. This step can be reached e.g. when the user terminates operation of the lighthead by giving through the user interface a shutdown command to the lighthead, by utilizing e.g. a shutdown switch/button.

The feedback arrows illustrated in the figure indicate a possible continuous/repeating nature of practicing the method. During a single working cycle, it is possible to adjust various light field features several times by an operator of the lighthead.

What has been described above represents just a few embodiments of the invention by way of example. The principle according to the invention can naturally be varied within the scope of protection defined by the claims, regarding for example implementation details as well as fields of use.

### Reference numerals

- 1: Lighthead
- 10: Lighthead support
- 2: Portable pointing device
- 20: Body
- 21: Processor
- 220: Memory (unit)
- 280: program for function button
- 282: program for light diode unit
- 284: program for battery
- 286: program for data transfer unit
- 288: program for position sensor
- 290: program for lighting unit
- 293: laser-controlling program
- 23: User input elements
- 24: Battery
- 25: Position sensor, acceleration sensor
- 26: Data transfer unit
- 27: Light diode unit
- 27a,: light source, light diode
- 27b: optics
- 271: light beam, pointing beam
- 29: Projection elements
- 29a: light source, laser
- 29b: optical film
- 291: instruction beam
- 22: Light diode unit
- 221: pointing beam (designation of light elements)
- 290: Computer program
- 3: Light elements
- 3¹, 3², 3³...: Light element
- 31: motion mechanisms for light elements
- 32: observation elements for light elements
- 4: Adjustment elements
- 400: Control system
- 440: control unit
- 442: data transfer unit
- 450: observation unit
- 5: Adjustment instructions
- 551, 552, 553: Adjustment patterns
- 551c, 552c, 553c: some adjustment patterns
- 551g, 552g, 553g: some adjustment patterns
- 512a, 512b, 512e, 513a: some adjustment symbols
- 511a, 511e, 511b: some adjustment symbols
- 6: Light field
- 600: Light field feature (brightness)
- 700: Light field feature (temperature)
- 8: Operating table
- 9: Object of surgical operation (patient)

## Claims

1. An operating room lighting system, which comprises a lighthead support (10) for a lighthead (1) with a plurality of light elements (3), especially LED light elements, for generating a light field on an object (9) of surgical operation to be illuminated on a patient, as well as adjustment elements (4) for controlling features of the lighting system, especially for controlling features of a light field generated by the lighthead (1), said adjustment elements (4) comprising a portable pointing device (2) for controlling the lighting system by means of a pointing beam (221) of said pointing device (2) by the action of a lighting system operator, **characterized in that** the portable pointing device (2) further comprises projection elements (29) for projecting, on or around a pointing beam (271) generated by the pointing device (2), adjustment instructions (5) relating to a feature of the lighting system, preferably to a feature of one or more light fields generated by the lighthead (1), or adjustment instructions (5) relating to a location of the light field, whereby the portable pointing device (2) has the projection elements (29) comprising at least one laser light source (29a), preferably a semiconductor laser, for projecting said adjustment instructions (5), as well as at least one light-transmitting optical film (29b) which is adapted to provide an adjustment instruction (5) from light generated by the at least one laser light source (29a), said optical film (29b) comprising diffractive optical microstructures such as surface-relief structures and/or embedded structures for producing the adjustment instructions (5).

2. A lighting system according to claim 1, **characterized in that** the location of adjustment instructions (5) around the pointing beam (271) of the pointing device (2) is determined on the basis of a mutual location of the projection elements (29) and the laser light source (29a) relative to each other.

3. A lighting system according to either of the preceding claims, **characterized in that** the optical film has on its surface a protective layer, such as a sheet or a glass or plastic layer.

4. A lighting system according to any of the preceding claims, **characterized in that** the adjustment instructions (5) are operating instructions, which relate to user input elements (23) and which consist of adjustment instruction patterns pertinent to light field features of the lighthead (1) adjustable or achievable with the user input elements (23) of the pointing device (2).

5. A lighting system according to any of the preceding claims, **characterized in that** the projection elements (29) are adapted to project the adjustment instructions (5) dynamically on the basis of each adjustable lighting system feature selected by a user with the pointing device (2), especially a feature of a light field generated by the lighthead (1).

6. A lighting system according to any of the preceding claims, **characterized in that** the projection elements (29) are adapted to project on or around the pointing beam (271) mutually different adjustment instructions for at least two adjustable features of a light field generated by the lighthead (1).

7. A lighting system according to any of the preceding claims, **characterized in that** the projected adjustment instructions (5) comprise graphic patterns or symbols.

8. A lighting system according to any of the preceding claims, **characterized in that** the projection elements (29) are adapted to project the adjustment instructions (5) as a regularly repeating pattern or structure on or around the pointing beam (271) generated by the pointing device.

9. A lighting system according to any of the preceding claims, **characterized in that** said portable pointing device (2) includes not only said projection elements (29) but also at least one processor (21) and at least one memory (22) comprising a computer program, at least one position sensor (25) for determining an orientation of the pointing device (2), a data transfer unit (26) for communicating data, especially position data, in a wireless manner to a control system (400) for the adjustment elements, as well as at least one user input element (23), such as a button, for receiving control commands issued by the user.

10. A lighting system according to claim 9, **characterized in that** the user input element (23) comprises a switch, button or touch-sensitive area, by turning, pressing, or touching of which it is possible to select or adjust a controllable light field feature of the lighting system, especially that of the lighthead.

11. A lighting system according to claim 1, **characterized in that** the lighting system adjustment elements (4) also comprise a control unit (440) for controlling the light elements (3), as well as observation elements (32) and/or an observation unit (450) for detecting a location of the portable pointing device in relation to the light elements (3).

12. A lighting system according to any of the preceding claims, **characterized in that** the optical film (29b) is a flexible polymer film.

13. A lighting system according to any of the preceding claims, **characterized in that** the optical film (29b) is less than 2mm, preferably less than 1 mm, and most preferably less than 0.5mm in thickness.

14. A lighting system according to any of the preceding claims, **characterized in that** the light field feature of the lighting system's lighthead (1), which is projectable with the projection elements (29), comprises at least one light field feature selected from the group of: the orientation of a light element's (3) longitudinal axis within a supply air stream of fresh air arriving by way of the ceiling, the orientation and focus of a light beam (30) emitted by a light element (3), the angle difference between a plane of the lighthead support and a plane extending by way of a site to be illuminated, the distance of the lighthead support (10) from ceiling structures, the orientation, brightness, color temperature, diameter, shape and size of a light field generated by the light element (3).

15. A lighting system according to claim 14, **characterized in that** the light field feature of the lighting system's lighthead (1), which is controllable with the projection elements, comprises at least one feature selected from the group of: the orientation and focus of a light beam emitted by a light element, the orientation, brightness, color temperature, diameter, shape and size of a light field.

16. A lighting system according to any of the preceding claims, **characterized in that** the projection elements (29) include a translatorily and/or rotatably movable, optionally motorized optical element, such as a mask, lens or mirror, for selecting at each time presentable adjustment instructions by way of a user interface on the basis of a user-indicated modifiable feature of the light field.

17. A lighting system according to any of the preceding claims, **characterized in that** the lighthead support comprises a plurality of light elements, which are fitted on mounting brackets and which comprise at least one light diode or LED, and said mounting brackets are arranged in the proximity of the ceiling of an operating room or the like facility, essentially above an operating table (8) so as to be located immediately below a supply air frame (TF) of the facility to be illuminated.

18. A method for presenting illumination adjustment instructions (5) to the operator of an operating room lighting system, said lighting system comprising a lighthead with a plurality of light elements (3), especially LED light elements, for generating a light field on an object (9) of surgical operation to be illuminated on a patient, as well as adjustment elements (4) for controlling features of the lighting system, especially for controlling features of a light field generated by the lighting system's lighthead, said adjustment elements (4) comprising a portable pointing device (2) for controlling the lighting system by means of a pointing beam (221) of said pointing device by the action of a lighting system operator, **characterized in that** the method comprises projecting adjustment instructions (5) relating to controlling features of the lighting system, especially projecting, on or around a pointing beam (271) generated by the pointing device (2), adjustment instructions relating to a change in one or more features of a light field generated by the lighting system's lighthead (1) or to a change in the light field's location, wherein the projecting is carried out by means of at least one laser light source (29a), preferably a semiconductor laser, and at least one light-transmitting optical film (29b) in such a way that the light of said laser light source is conducted through the optical film, preferably a diffractive film, for producing adjustment instruction patterns (5).

19. A method according to claim 18, **characterized in that** the method comprises at least the following steps of:
- indicating a to-be-illuminated or illuminated location (9) on an operating table (8) with the portable pointing device (2)
- selecting a feature of a light field generated by the lighthead (1) from a user input element (23) of the pointing device,
projecting, on or around a pointing beam (271) of the pointing device, an adjustment instruction (5) relating to a feature, or a change therein, of a light field generated by the lighthead, said pointing beam being present within the light field generated by the lighthead (1) or in a location to which the light field is to be relocated.

20. A method according to claim 19, which further comprises generating a pointing beam (271) with the portable pointing device (2) and/or generating a light field with the lighthead (1).

21. A method according to claim 19 or 20, **characterized in that** the location of a light field generated by the lighthead (1) or features of said light field are controlled by means of the pointing device (2) through the intermediary of a light beam or pointing beam (271) applied to light elements (3) of the lighthead (1)

22. A method according to any of claims 19-21, wherein the projecting proceeds dynamically on the basis of an each time adjustable, user-selected feature of the lighthead's (1) light field.

23. A method according to any of claims 19-22, wherein the adjustment instructions for a change in a feature of the light field are projected on or around the light field (271) of the pointing beam, said adjustment instructions relating to at least two mutually different features of the lighthead's (1) light field, which are capable of being adjusted.
